# EUROPEAN PATENT APPLICATION

(11) **EP 2 878 210 A1**
(43) Date of publication of application: **03.06.2015**
(21) Application number: 13822921.6
(22) Date of filing: 24.07.2013
(51) Int. Cl.: A23L 1/236, A23L 1/22

(54) **METHOD FOR PREPARING GALACTOSE FROM LARCH AND METHOD FOR PREPARING TAGATOSE USING GALACTOSE**

(30) Priority: 25.07.2012 KR 20120080975
(71) Applicant: CJ CheilJedang Corporation, Jung-gu Seoul 100-749 (KR)
(72) Inventor: YANG, Sung Jae, Gwangmyeong-si Gyeonggi-do 423-776 (KR); KIM, Min Hae, Incheon 407-050 (KR); LEE, Young Mi, Bucheon-si Gyeonggi-do 420-836 (KR); KIM, Yang Hee, Bucheon-si Gyeonggi-do 422-708 (KR); KIM, Jin Ha, Bucheon-si Gyeonggi-do 420-845 (KR); JEONG, Jae Hoon, Incheon 402-765 (KR); CHOI, Jong Min, Seoul 156-791 (KR); KIM, Seong Bo, Seoul 120-830 (KR); PARK, Seung Won, Yongin-si Gyeonggi-do 446-727 (KR)
(74) Representative: Nevant, Marc
(86) International application number: PCT/KR2013/006603
(87) International publication number: WO 2014/017814

(57) **Abstract**

The present invention relates to a method for preparing galactose, which is a key intermediate for preparation of tagatose, from larches. In addition, the present invention relates to a method for preparing tagatose through isomerization of the larch-derived galactose, and tagatose prepared by the method.

## Description

### Technical Field

The present invention relates to a method for preparing galactose from larches, a method for preparing tagatose from the larch-derived galactose, and tagatose prepared by the same.

### Background Art

Tagatose is an isomer of galactose and is known as one of natural low-calorie saccharides. Tagatose exhibits very similar sweetness to sugar, namely, is about 92% as sweet as sugar. However, tagatose has about 38% the calories of sugar while its glycemic index (GI) is only about 4% that of sugar. For these reasons, tagatose has been a highly favored sugar substitute.

Furthermore, tagatose has attained GRAS (Generally Recognized As Safe) status under the U.S. Food and Drug Administration, thereby permitting its use as a sweetener in foods, beverages, health foods, diet additives, and the like. Tagatose is known as having almost no side effects when digested in the body, and thus is expected to be widely used.

As methods for preparing tagatose in large amounts, there are chemical methods for preparing tagatose from galactose using a catalyst and a biological method for preparing tagatose through isomerization of galactose using an isomerase. The chemical methods require a high temperature and high pressure chemical process, which not only makes the method complicated but also causes low production rate and large amounts of industrial waste. In this regard, there is an increasing need for an enviromnentally friendly method for preparing tagatose.

As a biological method for preparing tagatose, a method for preparing tagatose by an enzymatic process through isomerization of galactose is known in the art. Galactose is a key intermediate material for preparation of tagatose and is generally obtained by lactose digestion (see Korean Patent Publication No. 10-1996-0014148A and the like as prior arts concerning a method for preparing galactose from lactose). However, the price of lactose on the global market varies wildly due to changes in amount of raw milk produced, demand for powdered milk, lactose consumption amount, and the like. Such price fluctuation makes it difficult to secure stable supply of the key intermediate for preparing tagatose.

Therefore, there is a need for a method for preparing large amounts of galactose for the food industry in a stable manner. Further, there is also a need for developing a method for preparing large amounts of tagatose using galactose.

### Technical Problem

It is an object of the present invention to provide a method for preparing a large amount of galactose which is a key intermediate for preparation of tagatose, in a stable manner.

It is another object of the present invention to provide a method for preparing tagatose through isomerization of the larch-derived galactose in an environmentally friendly, biological manner.

### Technical Solution

The present invention relates to a method for preparing galactose, which is a key intermediate for preparation of tagatose, from larches.

In addition, the present invention relates to a method for preparing tagatose through isomerization of the larch-derived galactose, and tagatose prepared by the same.

In accordance with one embodiment of the invention, a method for preparing galactose from larches includes: a) subjecting larches to hot water extraction to obtain arabinogalactan; b) performing acid hydrolysis of the hot water extracted arabinogalactan to obtain an acid hydrolysate; and c) separating galactose from the acid hydrolysate.

In accordance with another embodiment of the invention, in step a), the hot water extraction is performed using sliced larch or larch sawdust.

In accordance with a further embodiment of the present invention, in step a), the hot water extraction is performed at a temperature of 15°C to less than 100°C for 0.5 to 24 hours.

In accordance with yet another embodiment of the present invention, in step b), the acid hydrolysis includes adding 0.1% (wt/v) to 15% (wt/v) of sulfuric acid to the hot water extracted arabinogalactan.

In accordance with yet another embodiment of the present invention, in step c), the galactose separation includes separating galactose through chromatography.

In accordance with yet another embodiment of the present invention, the method for preparing galactose from larches further includes: performing decolorization and/or desalinization before or after c) separating galactose.

In accordance with yet another embodiment of the present invention, the method for preparing galactose from larches further includes performing decolorization and/or desalinization of the acid hydrolysate before c) separating galactose.

In accordance with yet another embodiment of the present invention, the acid hydrolysate is subjected to decolorization and/or desalinization.

In accordance with yet another embodiment of the present invention, the decolorization of the acid hydrolysate includes adding 0.1% (wt/v) to 10% (wt/v) of activated carbon to the acid hydrolysate.

In accordance with yet another embodiment of the present invention, the desalinization of the acid hydrolysate includes removing ion components from the acid hydrolysate using a cation exchange resin or an anion exchange resin.

In accordance with yet another embodiment of the present invention, the removing ion component includes using a cation exchange resin substituted with a hydrogen group or an anion exchange resin substituted with a hydroxyl group.

In accordance with yet another embodiment of the present invention, there is provided a method for preparing tagatose through isomerization of the larch-derived galactose.

In accordance with yet another embodiment of the present invention, isomerization is performed using *Corynebacterium* microbial cells having an arabinose isomerase producing ability.

In accordance with yet another embodiment of the present invention, the microbial cell includes *Corynebacterium glutamicum* CJ-1-TNAI (KCCM10786P).

In accordance with yet another embodiment of the present invention, there is provided tagatose prepared through isomerization of the larch-derived galactose.

In accordance with yet another embodiment of the present invention, there is provided tagatose prepared through isomerization of the larch-derived galactose using *Corynebacterium glutamicum* CJ-1-TNAI (KCCM10786P).

### Advantageous Effects

The present invention can provide a large amount of galactose in a stable manner using larches, which are naturally existing biomass as a raw material for galactose used as a key intermediate for preparation of tagatose.

Further, according to the present invention, tagatose, which is attracting keen attention as a sweetener for substituting sugar, can be prepared in a large amount at low cost using larches as a raw material in preparation of tagatose as mentioned above.

Furthermore, the preparation method of the present invention can enhance yield of galactose and tagatose and efficiency of the preparation process.

### Description of Drawings

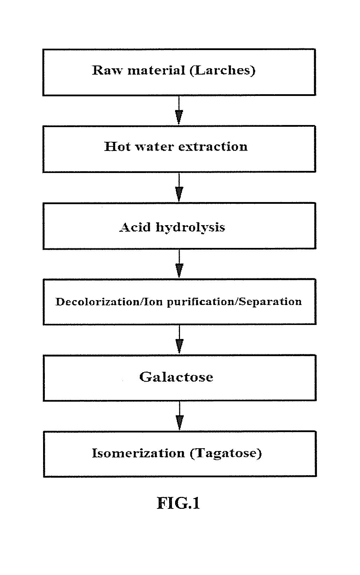

Fig. 1 shows a flowchart of a process for preparing tagatose by preparing galactose from larches and then preparing tagatose using galactose according to one embodiment of the present invention.
Fig. 2 shows HPLC chromatograms of glucose, xylose, galactose, arabinose and mannose.
Fig. 3 shows larch raw materials processed in various shapes before hot water extraction.
Fig. 4 shows an extraction yield of galactose according to extraction temperature.
Fig. 5 shows an extraction yield of galactose according to extraction time.

### Best Mode

Hereinafter, the present invention will be described in more detail. Descriptions of details apparent to those skilled in the art or in relevant fields will be omitted herein.

The present invention relates to a method for preparing galactose, which is a key intermediate for preparation of tagatose, from larches.

Further, the present invention relates to a method for preparing tagatose through isomerization of the larch-derived galactose, and tagatose prepared by the same.

Specifically, according to one embodiment of the invention, a method for preparing galactose from larches includes: a) subjecting larches to hot water extraction to obtain arabinogalactan; b) performing acid hydrolysis of the hot water extracted arabinogalactan to obtain an acid hydrolysate; and c) separating galactose from the acid hydrolysate.

Larches are deciduous coniferous trees and are classified in the division Gymnosperms, class *Coniferopsida*, order *Coniferales*, family *Pinaceae*. Unlike general pine, larches lose their leaves in autumn. Larches used in the present invention are not particularly limited, and any larch containing arabinogalactan may be used. Examples of larch may include *Larixsibirica* and *Larix gmelinii*, without being limited thereto.

Xylem of larches may be used in the present invention, and larches are not particularly limited by their place of production, age, growing weather, and the like. It is preferable to use larches with high content of saccharides in order to enhance yield of galactose. As a result of saccharide analysis of larches obtained in various places of production using high performance liquid chromatography (HPLC), *Larix Sibirica* is good in terms of saccharide content (a galactose content of about 20%).

Arabinogalactan is a water-soluble polysaccharide and consists of L-arabinose and D-galactose as main components. Arabinogalactan is abundant in a middle lamella of the cell wall of higher vegetables or xylem of conifers and the like. Arabinogalactan is generally present in a state of being coupled to a small amount (not more than 10%) of proteins.

In the present invention, in step a), hot water extraction refers to an extraction process performed by adding water to larches and heating the resulting mixture.

In order to increase extraction yield of arabinogalactan and galactose therefrom in hot water extraction, larches may be used after processing. Specifically, larches are used after being sliced into small slices. More specifically, larches are used after being processed into sawdust.

Although the small slices of larches are not particularly limited in terms of size or shape, the slices preferably have a size of 5 cm x 5 cm x 5 cm or less, and more preferably 1 cm x 1 cm x 1 cm or less. The size of the larch slices is inversely proportional to extraction yield.

In the event that hot water extraction is performed by processing larches into small slices or sawdust, extraction yield of arabinogalactan and galactose therefrom may be increased under the same extraction conditions, thereby improving process efficiency.

According to one embodiment of the invention, in order to improve extraction yield of arabinogalactan and galactose therefrom in hot water extraction, a preferable extraction temperature range is provided.

To improve extraction yield, hot water extraction is preferably formed at a temperature in the range of 15°C to less than 100°C, more preferably 30°C to less than 90°C, still more preferably 60°C to less than 80°C. Most preferably, hot water extraction is performed at a temperature in the range of 65°C to 75°C. Within this range of extraction temperature, good results can be obtained in terms of economic feasibility and extraction yield.

According to another embodiment of the invention, in order to improve extraction efficiency by taking into account extraction yield and economic feasibility of the extraction process in hot water extraction, a preferable extraction time range is provided.

In order to improve extraction yield, hot water extraction is preferably performed for 30 minutes to 6 hours, more preferably 30 minutes to 4 hours, still more preferably 30 minutes to 2 hours. Within this range of extraction time, good results can be obtained in terms of economic feasibility and extraction yield.

According to the present invention, although the number of extractions is not particularly limited, hot water extraction is performed once to three times, preferably once or twice, more preferably once. This is determined in consideration of economic feasibility since most arabinogalactan contained in larches is extracted by a primary hot water extraction stage.

According to the present invention, in step b), acid hydrolysis may include enriching arabinogalactan, performing acid hydrolysis of the concentrate to obtain an acid hydrolysate, and/or neutralizing the acid hydrolysate.

The operation of enriching arabinogalactan extracted from larches is not particularly limited and may be performed using any typical method used in the art or relevant field. For example, the enriching operation may be performed using an evaporator. The enriching operation is preferably performed until the concentrate has a sugar content of 10 Brix to 50 Brix, more preferably 20 Brix to 40 Brix.

The operation of performing acid hydrolysis of the arabinogalactan concentrate may be performed by adding an acid to the concentrate. More preferably, sulfuric acid is added to the concentrate, and then the resulting mixture is reacted in a high pressure sterilizer.

For acid hydrolysis, sulfuric acid is preferably added to the arabinogalactan concentrate in an amount of 1% (wt/v) to 15% (wt/v), more preferably 0.5% (wt/v) to 10% (wt/v), still more preferably 0.5% (wt/v) to 3% (wt/v). Within this range, acid hydrolysis of arabinogalactan can be effectively performed while securing a predetermined survival ratio of galactose (as calculated by dividing the concentration of galactose not decomposed by sulfuric acid after hydrolysis by the concentration of galactose prior to hydrolysis) and facilitating the neutralizing operation.

The operation of neutralizing the acid hydrolysate may be performed by adding a basic agent to the acid hydrolysate. For instance, calcium carbonate may be added. Neutralization is preferably performed to pH 6 to 8.

In the present invention, step c) of separating galactose may include separating galactose from the acid hydrolysate. Galactose separation may be performed by a separation method known in the art, such as fractional crystallization, chromatography, and the like. Chromatography is preferably used.

The method may further include performing decolorization and/or desalinization prior to or after step c) of separating galactose. Preferably, the method further includes decolorization and/or desalinization prior to step c) of separating galactose. Preferably, the method further includes decolorization and desalinization prior to step c) of separating galactose.

Decolorization is preferably performed by adding activated carbon to the arabinogalactan hydrolysate, followed by stirring and filtering the resulting materials. Preferably, the activated carbon is powdered activated carbon.

In decolorization, activated carbon is preferably added in a concentration of 0.1% (wt/v) to 10% (wt/v), more preferably 0.5% (wt/v) to 5% (wt/v), even more preferably 0.5% (wt/v) to 2% (wt/v). Within this range, it is possible to effectively decolorize the arabinogalactan hydrolysate.

In decolorization, the mixture of arabinogalactan hydrolysate and activated carbon is preferably stirred at a rate of 10 rpm to 100 rpm for 0.5 hours to 1 hour.

Removal of ion components is preferably performed using columns packed with a cation exchange resin and/or an anion exchange resin.

The cation exchange resin may be an exchange resin substituted with a hydrogen group and the anion exchange resin may be an exchange resin substituted with a hydroxyl group.

In case ion components are removed, chromatography for separating galactose and arabinose from the arabinogalactan hydrolysate may be effectively performed. Specifically, if ion components are present in large amounts in a material to be subjected to chromatography, active groups of the separation resin are substituted with the ion components, thereby deteriorating separation capability of the resin. For this reason, there is a problem that repetitive separation using the separation resin becomes difficult. Therefore, it is preferred to remove the ion components prior to chromatography.

The operations of enriching the decolorized and desalted hydrolysate and performing chromatography of the concentrate are preferably performed by passing the concentrate through an ion exchange resin substituted with a calcium group, followed by subjecting a solution eluted with deionized water to chromatography.

A further embodiment of the invention provides a method for preparing tagatose, which includes: preparing galactose from larches by the method disclosed in the present invention; and isomerizing the prepared galactose to obtain tagatose.

Isomerization is preferably performed using *Corynebacterium* microbial cells having an arabinose isomerase producing ability. The *Corynebacterium* microbial cell is a recombinant strain, more preferably *Corynebacterium glutamicum* CJ-1-TNAI (KCCM10786P) recombinant strain.

The *Corynebacterium glutamicum* CJ-1-TNAI was deposited on October 18, 2006 in the International Patent Organism Depositary, Korean Federation of Culture Collections affiliated Korea Culture Center of Microorganisms (361-221 Hongje 1-dong, Seodaemun-gu, Seoul, Korea), with a deposit number of KCCM10786P under the provisions of the Budapest Treaty. With regard to details of *Corynebacterium glutamicum* CJ-1-TNAI, see Korean Patent No. 10-0872694, which is incorporated herein by reference.

The *Corynebacterium glutamicum* CJ-1-TNAI is a recombinant strain prepared using a gene encoding an arabinose isomerase derived from *Thermotoga neapolitana*, which is an extreme thermophilic bacterium. The *Corynebacterium glutamicum* CJ-1-TNAI can produce an arabinose isomerase in a stable manner under conditions of high temperature and high concentration of galactose substrate, as compared to other strains, while mass cultured.

The method for preparing tagatose according to the present invention includes: a) culturing microbial cells having an arabinose isomerase producing ability and killing the microbial cells, b) mixing the killed microbial cells with alginate to prepare beads to which the killed microbial cells are immobilized, and c) introducing the larch-derived galactose to the prepared beads and isomerizing galactose into tagatose.

In step a), the culture of microbial cells having an arabinose isomerase producing ability is not particularly limited, and may be performed by any typical method used in the art.

Medium used in microbial cell cultivation is preferably a medium containing glucose, corn steeping liquid (CSL)), (NH₄)₂SO₄, KH₂PO₄, MgSO₄-7H₂O, kanamycin, and other components.

In step a), the operation of killing the microbial cells having an arabinose isomerase producing ability is not particularly limited, and preferably is performed by the following method.

In the operation of killing the microbial cells, a surfactant may be added and may include a cationic surfactant.

In the operation of killing the microbial cells, a surfactant is added to the microbial cells and then heated to 60°C to 90°C, more preferably 70°C to 75°C, for 0.5 hours to 2 hours to remove activity of the microbial cells.

The operation of killing the microbial cells under the conditions as set forth above is advantageous in that arabinose isomerase is not deteriorated while effectively killing the microbial cells, and thus can be sufficiently and effectively used.

In another embodiment of the invention, in step b), alginate is preferably an alginate solution prepared by dissolving alginate in a silicon dioxide solution.

The alginate solution is preferably prepared by dissolving 0.1 wt% to 5 wt% of alginate in 0.1 wt% to 3 wt% of the silicon dioxide solution. More preferably, the alginate solution is prepared by dissolving 1.5 wt% to 2.5 wt% of alginate in 0.4 wt% to 1.0 wt% of the silicon dioxide solution.

In step b), a syringe for preparing beads for injecting a mixed solution of the killed microbial cells and alginate is not particularly limited, and may include any syringe capable of being used in immobilization equipment (such as a bead production machine) commonly used in the art.

In a further embodiment of the invention, in step c), isomerization may be performed in a manner that the isomerization equipment is packed with the beads prepared in step b) and then galactose is introduced thereto.

Galactose may be a galactose solution prepared by dissolving galactose in a solution.

In the operation of introducing the galactose solution to the isomerization equipment packed with the beads, pH of the galactose solution is preferably adjusted to pH 7 to 8, and manganese chloride is introduced in an amount of 0.01 % 0.1 % to facilitate enzyme activation.

In yet another embodiment of the invention, after step c), the method for preparing tagatose further includes d) subjecting the isomerization solution in which galactose and tagatose are mixed to simulated moving bed chromatography.

Simulated moving bed chromatography refers to one of continuous separation processes for separating two materials having different degrees of affinity to an adsorbent packed in a separation column. In the simulated moving bed technique, instead of moving the adsorbent of simulated moving bed, a sample inlet position and a separated liquid collection position are moved stepwise to provide an impression of moving bed chromatography in which an adsorbent is actually moving.

In order to separate galactose and tagatose in step, calcium type cation resins preferably having a particle size of 200 µm to 400 µm, more preferably 220 µm to 320 µm, may be used.

Yet another embodiment of the invention provides tagatose prepared through isomerization of the larch-derived galactose.

### Mode for Invention

Hereinafter, the present invention will be described in more detail with reference to the following examples. It should be understood that these examples are provided for illustrative purposes only and are not to be construed in any way as limiting the present invention.

### Example 1

### Analysis of saccharide content in solution

In each step of a method of obtaining galactose from larches, the saccharide content in a solution of each step was measured using HPLC equipped with a refractive index detector.

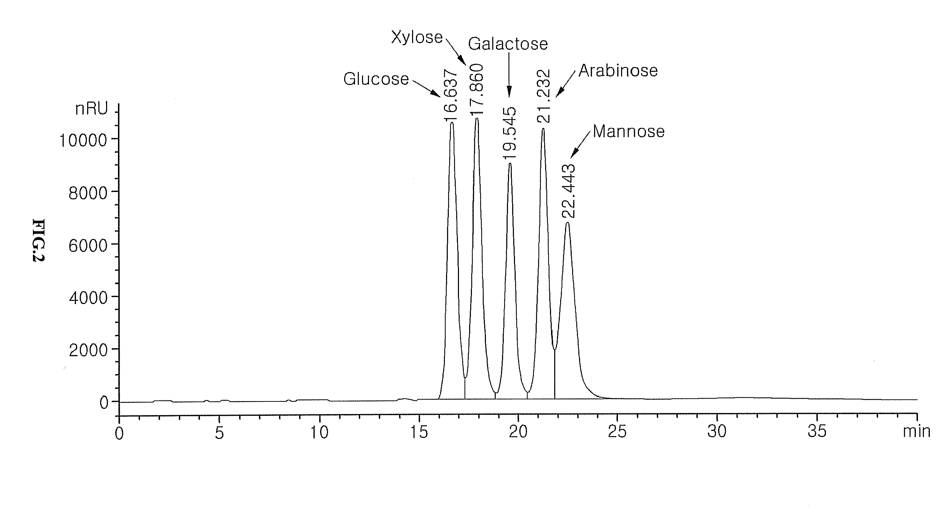

HPLC analysis was performed by injecting a sample into a column (SUPELCOGEL^{™} Pb column) set to 80°C, followed by passing distilled water as a mobile phase solvent at a rate of 0.3 ml/min therethrough. The standard samples were glucose, xylose, galactose, arabinose and mannose (Sigma-Aldrich), chromatograms of which are depicted in Fig. 2.

### Example 2

### Analysis of saccharide content in larches according to production place

In order to secure larches with a relatively high content of galactose, the saccharide content of larches according to production place was evaluated. The selected production places were Korea (Kangwon-province, Jeolla-province), Russia and China. In order to analyze the saccharide content in larches obtained from each production place, HPLC was employed. Analysis was performed under conditions as stated in Example 1. Results of analysis are shown in Table 1.

**TABLE 1**

| Place of production of larches | Galactose content (%) (on the basis of Ds) |
|---|---|
| Korea (Kangwon-province) | 9.22±0.89 |
| Korea (Jeolla-province) | 10.96±3.44 |
| Russia | 20.48±2.80 |
| China | 8.31±1.47 |

### Example 3

### Hot water extraction of arabinogalactan from larches

As a raw material for galactose, larches from Russia evaluated to have the highest galactose content in Example 2 were selected. In order to increase extraction yield of arabinogalactan and galactose therefrom and to optimize the extraction process, experiments were conducted by changing the shape of a larch raw material, extraction temperature, extraction time and number of extractions as major extraction conditions.

### Example 3-1: Extractions with different shapes of larch raw material

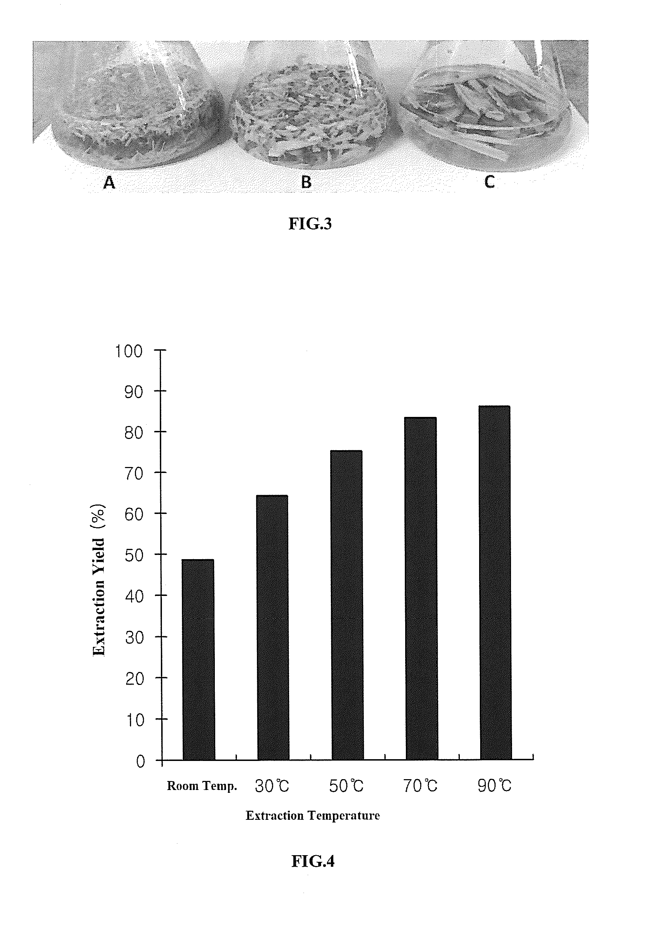

Larches from Russia were processed into three shapes, i.e. sawdust (A in Fig. 3), slices having a size of about 1 cm x 1 cm x 1 cm or less (B in Fig. 3) and slices having a size of about 1 cm x 5 cm x 1 cm or less (C in Fig. 3). The processed larches were subjected to hot water extraction.

Hot water extraction was performed by adding distilled water to each 20 g (based on dry weight) of larches processed into three shapes. For larch having a sawdust shape, 120 ml of distilled water was added, and for larches having a shape of slice of two sorts, 100 ml of distilled water was added for each slice. Hot water extraction was performed at 70°C with 24 hours of standing.

Results of hot water extraction are shown in Table 2.

**TABLE 2**

| Shape of raw material | Galactose extraction yield (%) |
|---|---|
| Sawdust | 93.0 |
| Slice having size of 1 cm x 1 cm x 1 cm or less | 85.9 |
| Slice having size of 1 cm x 5 cm x 1 cm or less | 73.4 |

From Table 2, it can be seen that a smaller larch raw material provides higher extraction yield.

### Example 3-2: Extraction with different extraction temperature

Larches from Russia were processed into a shape of sawdust as selected in Example 3-1 and subjected to extraction to confirm extraction yield according to extraction temperature. The experiment temperature was set to 15°C to 90°C. Extraction was performed by adding 120 ml of distilled water to 20 g (based on dry weight) of larch sawdust and standing for 24 hours.

The galactose extraction yield according to extraction temperature is shown in Fig. 4. Referring to Fig. 4, it can be confirmed that there is substantially no increase in galactose yield at an extraction temperature exceeding 70°C. Accordingly, the extraction temperature is preferably set to about 70°C by taking economics and changes in yield into account.

### Example 3-3: Extraction with different extraction time and number of extractions

Larches from Russia were processed into sawdust, followed by subjecting to hot water extraction by changing the extraction time and the number of extractions at 70°C.

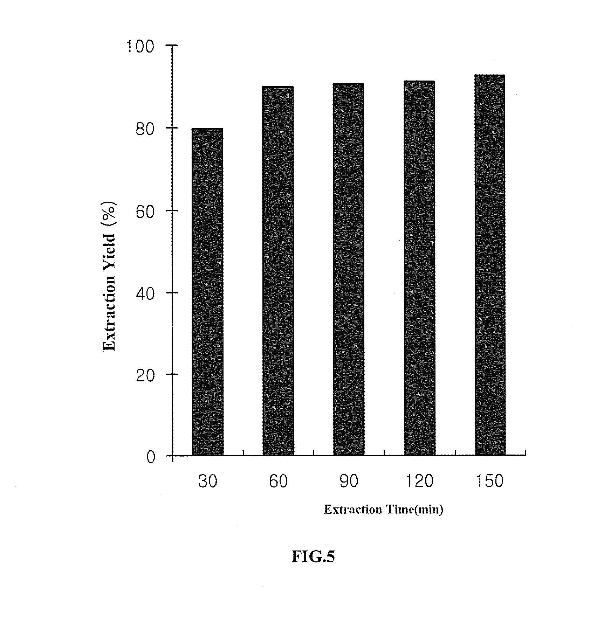

After adding 1.2L of distilled water to 200 g (dry weight) of larches in sawdust, extraction was performed at a time interval of 30 minutes for 5 hours while standing. Extraction yield according to extraction time is shown in Fig. 5.

Referring to Fig. 5 showing extraction yield according to extraction time, it can be confirmed that as the extraction time increases, yield and content of extracted material increase to a certain level, and after a certain time point (in case of this Example, after 2 hours), extraction yield remains almost constant. Since there is little increase in extraction yield after 2 hours of extraction, the extraction time is preferably set to about 30 minutes to about 2 hours by taking economics and changes in yield into account.

Fig. 5 shows results of primary extraction. By the primary extraction, it can be found that most of galactose contained in larches was extracted (equivalent to about 90% of total content). In order to further secure the remaining 10% of galactose which was not extracted, secondary extraction was performed under the same conditions as the primary extraction. As a result, an additional 5% of galactose was further harvested.

In conclusion, it is determined that extraction is preferably conducted only once by taking economics and yield into account.

### Example 4

### Acid hydrolysis of extracted arabinogalactan

In order to obtain galactose from arabinogalactan extracted from larches in Example 3, acid hydrolysis was performed.

Prior to acid hydrolysis, extracted arabinogalactan was enriched to 30 Brix using an evaporator. The arabinogalactan concentrate solution was divided into six samples, to which sulfuric acid was added in different concentrations (the sulfuric acid concentration in each sample was 0.5%, 1%, 1.5%, 2%, 3% and 10% (wt/v)). The resulting mixture was introduced into a high pressure sterilizer at 125°C, followed by reacting for 1 hour to facilitate hydrolysis.

After completion of hydrolysis, in order to perform neutralization, calcium carbonate was added to the acid hydrolysate to adjust pH to about 7. The neutralized hydrolysate was centrifuged at 8000 rpm to harvest the supernatant, which was then utilized in the next process. Further, the supernatant was analyzed by HPLC to evaluate the hydrolysis ratio.

The hydrolysis ratio of arabinogalactan and survival ratio of galactose according to sulfuric acid concentration are shown in Table 3. Herein, the survival ratio refers to a ratio calculated by dividing the concentration of galactose not digested with sulfuric acid after hydrolysis by the concentration of galactose prior to hydrolysis.

**TABLE 3**

| Concentration of sulfuric acid (% wt/v) | Hydrolysis ratio (%) | Survival ratio (%) |
|---|---|---|
| 0.5 | 96.6 | 89 |
| 1 | 96.7 | 88 |
| 1.5 | 96.7 | 86 |
| 2 | 97.14 | 82 |
| 3 | 98.2 | 76 |
| 10 | 100 | 46 |

At a concentration of 0.5% (wt/v) to 10% (wt/v) of sulfuric acid defined as the reaction condition, the hydrolysis ratios were almost similar, and the survival ratios showed tendency to decrease with increasing concentration of sulfuric acid. From the results, it was determined that a preferable concentration of sulfuric acid exhibiting high hydrolysis ratio while ensuring the survival ratio to some extent was about 0.5% (wt/v) to about 3 % (wt/v).

### Example 5

### Decolorization, ion purification, and chromatography of arabinogalactan hydrolysate

To the arabinogalactan hydrolysate prepared in Example 4, 0.5% (wt/v) to 2.0% (wt/v) of powdered activated carbon was added, followed by stirring at 10 rpm to 100 rpm for 0.5 hours to 1 hour, and then filtered through Whatman Filter paper 5 to remove colored materials.

In order to effectively separate galactose and arabinose from the arabinogalactan hydrolysate from which the colored materials were removed through chromatography, the decolored arabinogalactan hydrolysate was passed through columns packed with a cation exchange resin substituted with a hydrogen group and an anion exchange resin substituted with a hydroxyl group to remove ion components from the hydrolysate solution. Removal of the ion components was checked using a conductivity detector. It was determined that the ion components were removed when the conductivity was 10 microsiemens/cm or less.

The arabinogalactan hydrolysate from which impurities such as colored materials and ion components were removed was enriched to 60% (g/g solution), passed through an ion exchange resin (Amberlite CR1310 Ca) substituted with calcium. A solution eluted with deionized water was fractionated and subjected to chromatography, thereby harvesting a purified galactose solution.

### Example 6

### Preparation of tagatose through isomerization of larch-derived galactose

Using the larch-derived galactose prepared in Example 5, isomerization of galactose was performed in order to prepare tagatose.

In order to perform isomerization of galactose, *Corynebacterium glutamicum* CJ-1-TNAI (KCCM10786P) having an arabinose isomerase producing ability was used.

A culture medium was inoculated with *Corynebacterium glutamicum* CJ-1-TNAI (KCCM10786P) microbial cells at an initial concentration of O.D.₆₀₀ = 0.6, followed by culturing at 30°C for 20 hours. After cultivation, the cultured solution was centrifuged to collect microbial cells, which were suspended in distilled water until a ratio of the collected microbial cells was 20%. The suspended microbial cells of the recombinant strain were added to 2% (v/v) of an aqueous sodium alginate solution. The resulting mixed solution was dripped into a 100 mM CaCl₂ solution using a syringe pump and a vacuum pump to prepare microbial cell-alginate conjugates in which the microbial cells were trapped in sodium alginate beads.

A packed bed column (XK 26/40 column, Amersham Pharmacia) was filled with 100 ml of the recombinant strain immobilized on sodium alginate. Separately, a galactose solution as a substrate was adjusted to pH 7 using sodium hydroxide (NaOH), followed by introducing 0.06% of manganese chloride for enzyme activation, which was then passed through the isomerization column at a rate of 50 ml/hr at 55°C, thereby obtaining tagatose isomerized from galactose.

## Claims

1. A method for preparing galactose from larches, comprising:
subjecting larches to hot water extraction to obtain arabinogalactan;
performing acid hydrolysis of the hot water extracted arabinogalactan to obtain an acid hydrolysate; and
separating galactose from the acid hydrolysate.

2. The method for preparing galactose from larches according to claim 1, wherein the hot water extraction comprises processing larches into slices or sawdust.

3. The method for preparing galactose from larches according to claim 1, wherein the hot water extraction is performed at a temperature of 15°C to less than 100°C for 0.5 hours to 24 hours.

4. The method for preparing galactose from larches according to claim 1, wherein the acid hydrolysis comprises adding 0.1% (wt/v) to 15% (wt/v) of sulfuric acid to the hot water extracted arabinogalactan.

5. The method for preparing galactose from larches according to claim 1, wherein the galactose separation comprises separating galactose through chromatography.

6. The method for preparing galactose from larches according to claim 5, further comprising:
performing decolorization and/or desalinization of the acid hydrolysate before or after c) separating galactose.

7. The method for preparing galactose from larches according to claim 6, wherein the decolorization and/or the desalinization of the acid hydrolysate are performed before c) separating galactose.

8. The method for preparing galactose from larches according to claim 7, wherein the decolorization of the acid hydrolysate comprises adding 0.1% (wt/v) to 10% (wt/v) of activated carbon to the acid hydrolysate.

9. The method for preparing galactose from larches according to claim 7, wherein the desalinization of the acid hydrolysate comprises removing ion components from the acid hydrolysate using a cation exchange resin or an anion exchange resin.

10. The method for preparing galactose from larches according to claim 9, wherein the removing ion component is performed using a cation exchange resin substituted with a hydrogen group or an anion exchange resin substituted with a hydroxyl group.

11. A method for preparing tagatose, comprising:
preparing galactose from larches by the method according to any one of claims 1 to 10; and
isomerizing the prepared galactose.

12. The method for preparing tagatose according to claim 11, wherein isomerization is performed using *Corynebacterium* microbial cells having an arabinose isomerase producing ability.

13. The method for preparing tagatose according to claim 12, wherein the microbial cell comprises *Corynebacterium glutamicum* CJ-1-TNAI (KCCM10786P).

14. Tagatose prepared through isomerization of the larch-derived galactose.

15. Tagatose according to claim 14, wherein tagatose is prepared through isomerization of the larch-derived galactose using *Corynebacterium glutamicum* CJ-1-TNAI (KCCM10786P).
